# EUROPEAN PATENT SPECIFICATION

(11) **EP 2 173 941 B1**
(45) Date of publication and mention of the grant of the patent: **13.03.2013**
(21) Application number: 08762920.0
(22) Date of filing: 19.06.2008
(51) Int. Cl.: D21C 3/04, C12P 7/10, C13K 1/02, C12M 1/40, C12N 9/42, C12P 19/14

(54) **A SINGLE STEP PROCESS FOR SEPARATING BIOMASS COMPONENTS**
EINSTUFIGES VERFAHREN ZUR TRENNUNG VON BIOMASSEKOMPONENTEN
PROCÉDÉ À UNE SEULE ÉTAPE POUR SÉPARER DES COMPOSANTS DE BIOMASSE

(30) Priority: 20.06.2007 IN CH23782006
(43) Date of publication of application: 14.04.2010
(73) Proprietor: Nagarjuna Energy Private Limited, 500 082 Hyderabad (IN)
(72) Inventor: SRIVASTAVA, Suresh, Chandra, Hyderabad 500 082 (IN); SUDHAKARAN, Dinakaran, Samuel, Hyderabad 500 082 (IN); SARKAR, Manoj, Kumar, Hyderabad 500 082 (IN); PANDEY, Banibrata, Hyderabad 500 082 (IN); PECHIMUTHU, Sakthi, Priya, Hyderabad 500 082 (IN)
(74) Representative: Jones, Nicholas Andrew
(86) International application number: PCT/IB2008/001599
(87) International publication number: WO 2008/155634

(56) References cited:
- WO-A2-02/04084
- WO-A2-2006/086861
- DE-A1- 2 803 465
- GB-A- 2 040 332
- US-A- 4 941 944

## Description

### Field of the invention

The present invention relates to a method for fractionation of biomass components, more particularly, the present invention relates to a method for separating biomass into three major components such as lignin, cellulose, hemi-cellulose, wherein the cellulose thus obtained exist in a form which is amenable enzymatic saccharification.

### Background and Prior art

Lignocellulosic biomass must be treated to realize high yields, which is vital to commercial success in biological conversion. Better pre-treatment can reduce the use of expensive enzymes thus makes the process economically viable. Although many biological, chemical and physiological methods have been tried over the years, pre-treatment advances are still needed to reduce the overall cost.

A large number of literatures available on the use of organo-solvent to separate lignin, cellulose and hemicellulose from biomass, however, those technologies suffer from a major drawback. The cellulose recovered using the conventional processes, suffers from an inefficient saccharification and significant amount of cellulose loss as a degradation product. These leads to the higher process cost. In addition, cellulose either separated by any of the prior technologies requires huge enzyme loading or excess time to be get saccharified.

Till date all the existing processes involve organic solvents, water and acids to separate lignin, cellulose and hemi-cellulose but the Applicant with their best effort could not find any references that disclose a use of a catalyst to increase the efficiency of downstream process i.e. subsequent saccharification of cellulose. Briefly, the conventional processes, the lignin recovery and hemicellulose hydrolysis efficiency is not high and the cellulose obtained suffers from inefficient enzymatic saccharification.

### Objects of the invention

The primary object of the present invention is to develop a process to separate cellulose, hemicellulose and lignin with high purity and yield.

Another object of the invention is to obtain cellulose in such a form that subsequent saccharification becomes highly efficient thereby render downstream saccharification process economically viable.

Yet another object of the present invention is to provide a reactor to perform the instant method.

### Brief description of figure:

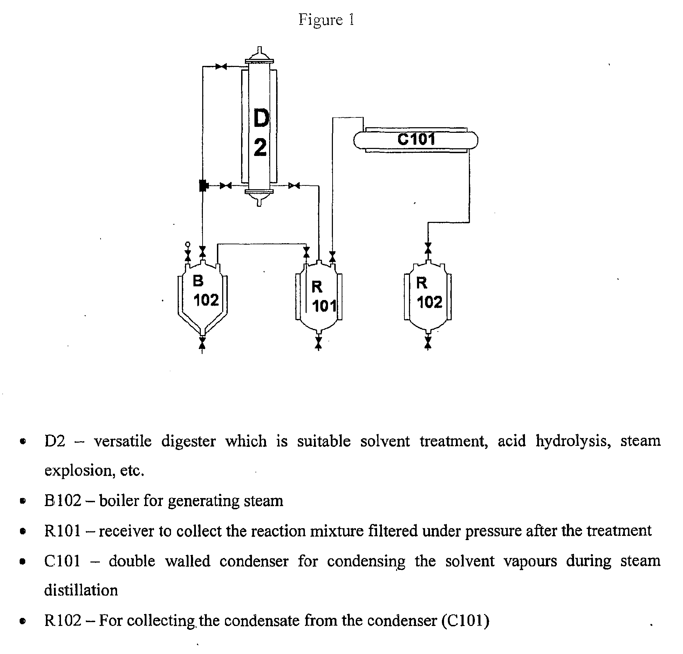

Figure 1 illustrates a system for obtaining cellulose from biomass in accordance of an embodiment of the present invention.

### Brief description of the Tables:

- Table 1: Shows the role of catalyst in preventing the cellulose loss during the process. In the absence of catalyst cellulose loss was more than 30% and this loss was reduced to about 5% when the catalyst was used.
- Table 2: Susceptibility of cellulose obtained by the instant process to enzymatic saccharification. The Table clearly indicates that the obtained cellulose is amenable to almost complete saccharification within 24 hours.
- Table 3: Compares the absorption bands of lignin obtained using instant process with the bands of pure lignin as reported in literature.

### Detailed description of the present invention:

Accordingly, the present invention provides a process for separating lignin, cellulose and 'hemi-cellulose and cellulose thus obtained from biomass in a form amenable to complete enzymatic hydrolysis. A process for obtaining cellulose from biomass in a form amenable to complete enzymic hydrolysis is described in claim 1.

In an aspect of the present invention, the water immiscible solvent selected from a group comprising higher alcohols like butanol, iso-amyl alcohol.

In another aspect of the present invention, the concentration of the water immiscible organic solvent in the reaction mixture is in the range of 40% to 80%.

In still another aspect of the present invention, the acid is mineral acid.

The metal salt catalyst selected from a group comprising copper sulphate, ferrous sulphate, Ferrous ammonium sulphate, Nickel sulphate, Sodium sulphate, ferric chloride.

In a further aspect of the present invention, the acid concentration is about 1% (v/v).

The catalyst concentration is in the range of 0.1% to 3% (w/v)

In yet another aspect of the present invention the process is carried out for a period of 10 to 30 min.

The present invention also provides a system for obtaining cellulose from biomass as defined in amended claim 8. Described is
a) a reactor chamber containing biomass and a mixture of an organic solvent immiscible in water, an acid, and a metal salt catalyst as defined below, having (i) a first inlet for supplying mixture of an organic solvent, (ii) a second inlet and (iii) at least one outlet;
b) a boiler in fluid flow communication with the second inlet of the reactor chamber for supplying steam to the reactor chamber,
c) a receiver coupled to the outlet of the reactor chamber for receiving hydrolysate from reactor chamber;
d) a steam distillation assembly for the removing traces of solvent in the aqueous fraction and precipitating the lignin in the solvent fraction.

In an aspect of the present invention, the first inlet of the reactor chamber is in fluid flow communication with a vessel, which contains mixture of an organic solvent.

In another aspect of the present invention, the receiver is in fluid flow communication with the boiler.

In one more aspect of the present invention, the steam distillation assembly comprises a condenser and a receiver for collecting the condensate from the condenser.

Accordingly, the present invention discloses a process of separating various components of biomass such as lignin, cellulose and hemicellulose with higher yield.

One of the preferred aspect of the instant process is to obtain cellulose in a form, which is highly amenable to enzymatic degradation.

One more advantageous aspect of the present invention is that the process involves a single step process thereby reduce the energy involvement.

The instant process comprising, contacting the biomass with a mixture of an organic solvent immiscible in water, a mild acid and a catalyst as defined dissolved in the acid solution a temperature in the range 120° - 220°C and pressure in the range 1.5-20 Bar and then filtering under pressure to separate the dissolved lignin, hydrolyzed hemicellulose and leaving behind a residue rich in cellulose, wherein the separated lignin and hemicellulose is in the solvent and aqueous phase respectively.

The present process efficiently degraded lignocellulosic biomass such as sweet sorghum bagasse, rice straw, wheat straw, sugar cane bagasse, corn stover, miscanthus, switch grass and various agricultural residues. Preferably, the materials are comminuted into particles before treatment.

In one or more aspects of the process, lignocellulosic biomass is treated with a mixture of a water immiscible solvent, preferably butanol, a mild acid and an additional catalyst as defined dissolved in the acid to dissolve a major portion of the lignin, hydrolyze the hemicellulose and obtain a residue that is rich in cellulose, which is highly reactive.

The present process utilizes a mixture of a water immiscible solvent, a mild acid and a defined water soluble metal salt as catalyst. The ratio of solvent to acidic water is 40:60 to 80:20 and preferably 60:40 and wherein the water contains not more than 1% acid. Further, the concentration of the catalyst dissolved in the water is in the range of 0.1 % to 3 wt %.

The water immiscible solvent used is preferably an aliphatic alcohol with at least 4 carbon atoms preferably butanol.

The water soluble metal catalyst is selected from copper sulphate, ferrous sulphate, ferrous ammonium sulphate, Nickel sulphate, Sodium sulphate and Ferric chloride.

The digestion is carried out at an elevated temperature and pressure. The digestion mixture is heated in the reactor to a temperature in the range of about 120°C to 220°C preferably for a period in the range of 10 to 30 minutes. The pressure maintained during digestion is in the range of 1.5 Bar to 20 Bar and preferably 15 Bar.

The lignocellulosic biomass, organic solvent immiscible in water, acidic water and metal salt catalyst taken in reactor, wherein the solid loading of biomass is about 15% with respect to the liquid. The reactor heated to raise the predetermined temperature by direct steam injection from the boiler. After holding for preferably 10 min in the desired condition, the reaction mixture filtered under pressure. The filtrate separated into two phases, the organic phase contains the lignin dissolved in it and the aqueous phase has the hemicellulose in the form of pentose sugars. The residue left behind in the reactor is rich in cellulose.

The lignin dissolved in the solvent can be easily recovered by a simple steam distillation process and the lignin obtained thereby is its native form.

The hemicellulose fraction, which obtained as pentose sugars in the aqueous fraction, has minimal sugar degradation products.

The residue obtained is rich in reactive cellulose, which is evident from its susceptibility to enzymatic saccharification.

The process of the present invention can be performed by a system for obtaining cellulose from biomass as shown in figure 1.

As can be observed from figure 1 the system of the present invention comprises a reactor chamber in which the biomass to be treated is contained. The reactor chamber is shown in figure 1 as versatile digester (D2) which is suitable for solvent treatment, acid hydrolysis, steam explosion, etc. Said reactor chamber has at least one inlet and at least one outlet. In a preferred embodiment of the present invention, the reactor chamber is having first inlet, second inlet and at least one outlet. The first inlet can be used for supplying mixture of an organic solvent. The second inlet can be used for supplying steam to the reactor chamber. A boiler (B102) for generating steam and supplying the same to the reactor chamber is in fluid flow communication with the second inlet of the reactor chamber. A first receiver (R101) coupled to the outlet of the reactor chamber for receiving the hydrolysate; Said first receiver can also be connected with the boiler for subsequent operation.

A steam distillation assembly for the removing traces of solvent in the aqueous fraction and precipitating the lignin in the solvent fraction. The steam distillation assembly comprises a condenser and a second receiver (R102). The condenser is in flow communication with the first receiver (R101) and provides the outlet to the second receiver (R102).

### Examples

A further description of the invention is given in examples below, which should not however be construed to limit the scope of the present invention.

### Example 1:

### Effect of catalyst on the process

100g of sweet sorghum bagasse added to the reactor. To this bagasse 60% butanol in 1% sulphuric acid for control run and 60% butanol in 1% sulphuric acid having either 0.5 mmol copper sulphate or ferrous ammonium sulphate or ferrous sulphate dissolved in it added to give a solid concentration of 15%. The reactor then heated to 160°C with live steam injection. The contents held at that temperature for 10 min, after that the contents filtered under pressure.

The filtrate separated out into two layers, the aqueous layer was steam distilled and then the sugars dissolved analyzed. The solvent fraction was steam distilled and the lignin obtained as a residue. The residue remaining in the reactor analyzed for cellulose, hemicellulose and lignin. The results are given in table 1. As it is evident from the table that with the use of water soluble metal catalyst in the reaction media reduces the cellulose loss minimized to nearly 5% and significant amount of hemicellulose and lignin separated.

**Table 1**

| **Catalyst** | **% loss of biomass component in separation process** | | |
|---|---|---|---|
| | **Cellulose** | **Hemicellulose** | **Lignin** |
| Control without catalyst | 30.42% | 79.38% | 67.51% |
| 0.5 mmol copper sulphate | 6.41% | 87.74% | 68.66% |
| 0.5 mmol ferrous ammoniaum sulphate | 8.55% | 82.04% | 51.81% |
| 0.5 mmol ferrous sulphate | 5.04% | 79.98% | 58.27% |

### Example 2:

### Susceptibility of pretreated residue to enzymatic saccharification

A 10% slurry of solid residues obtained as in experimental run of Example 1* saccharified with commercial cellulase enzyme preparation at 60 FPU/g of the enzyme loading. The contents incubated at 50°C at a pH of 4.5 for a period of 24 hrs. After the incubation time, sugars analyzed to estimate the saccharification percentage. The saccharification results in Table 2 clearly indicate the susceptibility of the pretreated residue to the cellulase enzyme. It can be concluded that the obtained cellulose is amenable to complete saccharification within 24 hours. Cellulose obtained using any conventional process the saccharification time is several days.

**Table 2**

| **Sample** | **% saccharification** | |
|---|---|---|
| | **In terms of glucose** | **In terms of reducing sugars** |
| 60% BuOH with 0.5 mmol CuSO₄/160 °C | 100.0% | 100.0% |
| 60% BuOH with 0.5 mmol FAS*/160 °C | 72.7% | 85.2% |
| 60% BuOH with 0.5 mmol FeSO₄/160 °C | 78.4% | 84.5% |
| 60% BuOH with 2g CuSO₄/180 °C | 75.1% | 99.9% |
| 60% BuOH with 2g FAS*/180 °C | 90.8% | 92.35% |
| 60% BuOH with 2g FeSO₄/180 °C | 96.1% | 99.2% |
| 80% BOH with 2g CuSO₄/ 180°C | 91.6% | 94.2% |

| | | |
|---|---|---|
| * *FAS-Ferrous ammonium sulphate* | | |

### Example 3:

### Characterization of solvent treated lignin

The lignin obtained from the solvent fractions characterized by FTIR analysis. The results indicate the lignin obtained in the present process is comparable to pure lignin reported in literature (Table 3).

**Table 3**

| **Reported in literature*** | | **Present Invention Absorption bands** |
|---|---|---|
| **Absorption bands** | **Assignment** | |
| 3429 | OH stretching | 3405 |
| 2945 | OH stretching of methyl or methylene or methane group | 2926 |
| 1732, 1726 | C=O stretch in un-conjugated ketone and carboxyl group | 1701 |
| 1660, 1653 | C=O stretch in conjugated ketone | - |
| 1606 | Aromatic skeletal vibrations | 1602 |
| 1507 | Aromatic skeletal vibrations | 1513 |
| 1460 | Aromatic methyl group vibrations | 1460 |
| 1434 | Aromatic skeletal vibrations | 1425 |
| 1374 | Aliphatic C-H stretch in CH₃ | - |
| 1328 | Syringyl ring breathing with C-O stretching | 1328 |
| 1242 | Aromatic C-O stretch | 1266 |
| 1165 | C-O stretchs in ester groups | 1165 |
| 1135 | Aromatic C-H in - plane deformation for syringyl type | 1122 |
| 1043 | Aromatic C-H in - plane deformation for guaiacyl type | 1032 |
| 855, 844 | Aromatic C-H out - plane bending | 832 |

| | | |
|---|---|---|
| ***** Source - F. Xu et al. / Industrial Crops and Products 23 (2006) 180-193 | | |

## Claims

1. A process for obtaining cellulose from biomass in a form amenable to complete enzymatic hydrolysis, said process comprising contacting the biomass with:-
a mixture of an organic solvent immiscible in water,
an acid, and
a metal salt catalyst
dissolved in the acidic water solution
at a temperature in the range of 120° - 220°C and a pressure in the range of 1.5 - 20 Bar and filtering the reaction mixture under pressure to separate the dissolved lignin, hemicellulose in the solvent and aqueous phase respectively and leaving behind pure cellulose,
wherein the metal salt catalyst is selected from copper sulphate, ferrous sulphate, Ferrous ammonium sulphate, Nickel sulphate, Sodium sulphate and ferric chloride, and wherein the catalyst is present in the range of 0.1% to 3% (w/v) of acidic water.

2. A process as claimed in claim 1, wherein the water immiscible solvent is selected from a group comprising higher alcohols.

3. A process as claimed in claim 2, wherein the higher alcohols is selected from the group comprising butanol, iso-amyl alcohol.

4. A process as claimed in any preceding claim, wherein the solvent to acidic water ratio is in the range of 40:60 to 80:20.

5. A process as claimed in any preceding claim, wherein the acid is a mineral acid.

6. A process as claimed in claim 5, wherein the acid concentration is about 1% (v/v) of water.

7. A process as claimed in any preceding claim, wherein the process is carried out for a period of time of 10 to 30 min.

8. A system for obtaining cellulose from biomass in a form amenable to complete enzyme hydrolysis, comprising:
(a) a reactor chamber (D2) containing biomass and
a mixture of an organic solvent immiscible in water,
an acid, and
a metal salt catalyst selected from copper sulphate, ferrous sulphate, ferrous ammonium sulphate, nickel sulphate, sodium sulphate and ferric chloride, wherein the catalyst is present in the range of 0.1% to 3% (w/v) of acidic water,
the reactor chamber having (i) a first inlet for supplying mixture of the organic solvent, (ii) a second inlet and (iii) at least one outlet;
(b) a boiler (B102) in fluid flow communication with the second inlet of the reactor chamber for supplying steam to the reactor chamber;
(c) a receiver (R101) coupled to the outlet of the reactor chamber for receiving filtered hydrolysate from reactor chamber;
(d) a steam distillation assembly (C101; R102) for removing traces of solvent in the aqueous fraction and precipitating the lignin in the solvent fraction.

9. A system for obtaining cellulose from biomass as claimed in claim 8, wherein the first inlet of the reactor chamber (D2) is in fluid flow communication with a vessel which contains mixture of an organic solvent.

10. A system for obtaining cellulose from biomass as claimed in claim 8 or claim 9, wherein the receiver (R101) is in fluid flow communication with the boiler (B102).

11. A system for obtaining cellulose from biomass as claimed in any of claims 8-10, wherein the steam distillation assembly (C101; R102) comprises a condenser (C101) and a receiver (R102) for collecting the condensate from the condenser.

## Patentansprüche

1. Verfahren zum Gewinnen von Cellulose aus Biomasse in einer Form, die einer vollständigen enzymatischen Hydrolyse zugänglich ist, wobei das Verfahren das Kontaktieren der Biomasse mit:
einer Mischung aus einem organischen Lösungsmittel, das nicht mit Wasser mischbar ist,
einer Säure und
einem Metallsalzkatalysator,
gelöst in der sauren Wasserlösung,
bei einer Temperatur im Bereich von 120° bis 220°C und einem Druck im Bereich von 1,5 bis 20 bar und Filtern der Reaktionsmischung unter Druck, so dass jeweils das gelöste Lignin, Hemicellulose in dem Lösemittel und wässrige Phase abgetrennt und reine Cellulose zurückgelassen wird,
wobei der Metallsalzkatalysator aus Kupfersulfat, Eisen-(II)-sulfat, Ferroammoniumsulfat, Nickelsulfat, Natriumsulfat und Eisen-(III)-chlorid ausgewählt ist und wobei der Katalysator in einem Bereich von 0,1 % bis 3 % (w/v) von saurem Wasser vorhanden ist.

2. Verfahren nach Anspruch 1, bei dem das mit Wasser unmischbare Lösungsmittel aus einer Gruppe ausgewählt ist, die höhere Alkohole umfasst.

3. Verfahren nach Anspruch 2, bei dem die höheren Alkohole aus der Gruppe ausgewählt sind, die Butanol, Isoamylalkohol umfasst.

4. Verfahren nach einem der vorherigen Ansprüche, bei dem das Verhältnis von Lösungsmittel zu saurem Wasser im Bereich von 40:60 bis 80:20 liegt.

5. Verfahren nach einem der vorherigen Ansprüche, bei dem die Säure eine Mineralsäure ist.

6. Verfahren nach Anspruch 5, bei dem die Säurekonzentration etwa 1 % (v/v) von Wasser beträgt.

7. Verfahren nach einem der vorherigen Ansprüche, bei dem das Verfahren eine Zeitdauer von 10 bis 30 min lang ausgeführt wird.

8. System zum Gewinnen von Cellulose aus Biomasse in einer Form, die einer vollständigen enzymatischen Hydrolyse zugänglich ist, umfassend:
(a) eine Reaktorkammer (D2), enthaltend Biomasse und
eine Mischung aus einem organischen Lösungsmittel, das nicht mit Wasser mischbar ist,
einer Säure und
einem Metallsalzkatalysator, ausgewählt aus Kupfersulfat, Eisen-(II)-sulfat, Ferroammoniumsulfat, Nickelsulfat, Natriumsulfat und Eisen-(III)-chlorid, wobei der Katalysator in einem Bereich von 0,1 % bis 3 % (w/v) von saurem Wasser vorhanden ist,
wobei die Reaktorkammer (i) einen ersten Einlass zur Zuführung der Mischung des organischen Lösungsmittels, (ii) einen zweiten Einlass und (iii) wenigstens einen Auslass aufweist,
(b) einen Dampferzeuger (B102) in Fließverbindung mit dem zweiten Einlass der Reaktorkammer, zur Zuführung von Dampf zur Reaktorkammer,
(c) ein Sammelgefäß (R101), das mit dem Auslass der Reaktorkammer verbunden ist, zur Aufnahme der gefilterten Hydrolysate aus der Reaktorkammer,
(d) eine Dampfdestillationsanordnung (C101; R102) zur Entfernung von Lösungsmittelspuren in der wässrigen Fraktion und Ausfällen des Lignins in der Lösungsmittelfraktion.

9. System zum Gewinnen von Cellulose aus Biomasse nach Anspruch 8, bei dem der erste Einlass der Reaktorkammer (D2) in Fließverbindung mit einem Behälter steht, der die Mischung des organischen Lösungsmittels enthält.

10. System zum Gewinnen von Cellulose aus Biomasse nach Anspruch 8 oder Anspruch 9, bei dem das Sammelgefäß (R101) in Fließverbindung mit dem Dampferzeuger (B102) steht.

11. System zum Gewinnen von Cellulose aus Biomasse nach einem der Ansprüche 8 bis 10, bei dem die Dampfdestillationsanordnung (C101; R102) einen Kühler (C101) und ein Sammelgefäß (R102) zur Aufnahme des Kondensats aus dem Kühler umfasst.

## Revendications

1. Procédé d'obtention de cellulose à partir de biomasse dans une forme susceptible de compléter l'hydrolyse enzymatique, ledit procédé comprenant la mise en contact de la biomasse avec
un mélange d'un solvant organique non miscible avec l'eau,
un acide, et
un sel métallique comme catalyseur
dissolus dans la solution d'eau acide
à une température comprise entre 120° et 220°C et à une pression comprise entre 1,5 et 2,0 bar et le filtrage du mélange réactionnel sous pression pour séparer respectivement la lignine dissolue, l'hémicellulose dans le solvant et une phase aqueuse et laisser la cellulose pure,
dans lequel le sel métallique servant de catalyseur est sélectionné parmi du sulfate de cuivre, du sulfate ferreux, du sulfate ferroso-ammoniacal, du sulfate de nickel, du sulfate de sodium et du chlorure ferrique et dans lequel le catalyseur est présent dans la plage entre 0,1% et 3% (poids/volume) d'eau acide.

2. Procédé suivant la revendication 1, dans lequel le solvant non miscible avec l'eau est sélectionné parmi un groupe comprenant des alcools supérieurs.

3. Procédé suivant la revendication 2, dans lequel les alcools supérieurs sont sélectionnés parmi le groupe constitué de butanol, d'alcool isoamylique.

4. Procédé suivant une quelconque des revendications précédentes, dans lequel le rapport solvant/eau acide se situe dans la plage comprise entre 40/60 et 80/20.

5. Procédé suivant une des revendications précédentes, dans lequel l'acide est un acide minéral.

6. Procédé suivant la revendication 5, dans lequel la concentration d'acide est de 1 % pourcent/volume d'eau environ.

7. Procédé suivant une des revendications précédentes, le procédé étant exécuté pendant une durée de 10 à 30 minutes.

8. Système permettant l'obtention de cellulose à partir de biomasse dans une forme susceptible de compléter l'hydrolyse enzymatique, comprenant
(a) une chambre réactionnelle (D2) contenant de la biomasse et
un mélange d'un solvant organique non miscible avec l'eau,
un acide et
un sel métallique comme catalyseur sélectionné parmi un sulfate de cuivre, un sulfate ferreux, un sulfate ferroso-ammoniacal, un sulfate de nickel, un sulfate de sodium et un chlorure ferrique, dans lequel le catalyseur est présent dans la plage entre 0,1 % et 3% poids/volume d'eau acide,
la chambre réactionnelle ayant (i) une première entrée permettant l'alimentation en mélange de solvant organique (ii), une seconde entrée (iii) et au moins une sortie,
(b) une chaudière (B102) qui communique par écoulement fluidique avec la seconde entrée de la chambre réactionnelle afin d'alimenter celle-ci en vapeur,
(c) un récipient (R101) couplé à la sortie de la chambre réactionnelle pour recevoir l'hydrolysat filtré provenant de celle-ci,
(d) un ensemble de distillation de vapeur (C1901, R102) pour enlever les traces de solvant dans la fraction aqueuse et précipiter la lignine dans la fraction de solvant.

9. Système permettant l'obtention de cellulose à partir de biomasse selon la revendication 8, dans lequel la première entrée de la chambre réactionnelle (D2) communique par écoulement fluidique avec un réservoir qui contient le mélange de solvant organique.

10. Système permettant l'obtention de cellulose à partir de biomasse selon la revendication 8 ou 9, dans lequel le récipient (R101) communique par écoulement fluidique avec la chaudière (B102).

11. Système permettant l'obtention de cellulose à partir de biomasse suivant une quelconque des revendications 8 à 10, dans lequel l'ensemble de distillation de vapeur (C101, R102) comprend un condensateur (C101) et un récipient (R102) pour collecter le produit de la condensation provenant du condensateur.
